# EUROPÄISCHE PATENTSCHRIFT

(11) **EP 0 485 374 B1**
(45) Veröffentlichungstag und Bekanntmachung des Hinweises auf die Patenterteilung: **07.04.1993**
(21) Anmeldenummer: 89908171.5
(22) Anmeldetag: 17.07.1989
(51) Int. Cl.: A61F 13/02, A61L 15/16

(54) **VERBANDMATERIAL MIT EINER MIKROPORÖSEN KUNSTSTOFFTRÄGERSCHICHT UND EINER DARAUF AUFGEBRACHTEN FLÜSSIGKEITSAUFNEHMENDEN SCHICHT**
DRESSING COMPRISING A MICROPOROUS PLASTIC BASE LAYER AND A LIQUID-ABSORBANT LAYER APPLIED THERETO
PANSEMENT AVEC UNE COUCHE DE BASE MICROPOREUSE EN PLASTIQUE RECOUVERTE D'UNE COUCHE ABSORBANT LES LIQUIDES

(43) Veröffentlichungstag der Anmeldung: 20.05.1992
(73) Patentinhaber: Lohmann GmbH & Co. KG, 56567 Neuwied (DE)
(72) Erfinder: THEILEMANN, Horst, A., D-8000 München 21 (DE)
(74) Vertreter: Flaccus, Rolf-Dieter, Dr.
(86) Internationale Anmeldenummer: EP8900833
(87) Internationale Veröffentlichungsnummer: WO9101122

(56) Entgegenhaltungen:
- EP-A-01 843 92
- EP-A-02 724 92
- DE-A-31 113 36
- US-A-48 030 78

## Beschreibung

Die Erfindung bezieht sich auf ein Verbandmaterial mit einer für Gas und Wasserdampf durchlässigen, für Wasser undurchlässigen mikroporösen Kunststoffträgerschicht und einer darauf aufgebrachten flüssigkeitsaufnehmenden Schicht.

Verbandmaterialien mit einem mindestens zweischichtigen Aufbau zur Behandlung offener Wunden sind schon lange bekannt. Sie bestehen in der Regel aus einer trockenen flüssïgkeitsaufnehmenden Schicht, die aus der Wunde sickernde Sekrete aufnehmen soll, und einer zweiten Schicht, die über die eigentliche Wundabdeckung übersteht und auf diesem überstehenden Anteil üblïcherweise mit einer Klebemasse versehen ist. Diese zweite Schicht ist entweder nicht wasserabweisend oder aber zur Gewährleistung einer ausreichenden Sauerstoffzufuhr mit bloßem Auge erkennbaren, einen Feuchtigkeitsdurchtritt in beide Richtungen gestattenden Poren versehen. Dieser Durchtritt von Feuchtigkeit kann einerseits bei Kontakt mit wäßrigen Lösungen von außen zu einer unerwünschten Befeuchtung der Wunde führen, oder aber andererseits zu einem Durchtritt von Blut oder Wundsekret zur körperabgewandten Oberfläche des Pflasters, so daß unter Umständen die Kleidung oder die Bettwäsche befeuchtet oder verschmutzt wird.

Vor allem bewirkt der Flüssigkeitstransport von der Wunde zur Wundoberfläche einen Feuchtigkeitsverlust des Körpergewebes im Wundbereich, was insbesondere bei Brandwunden nachteilig für die Heilung ist. Zum anderen tritt nach der Durchtränkung des Verbandes mit Blut bzw. Wundsekret ein Austrocknen an der Außenseite des Trägergewebes und damit ein Antrocknen des Verbandes an die Wunde ein. Beim Entfernen des Verbandes wird die Wunde schmerzhaft aufgerissen und das Abheilen verzögert.

Ein Verbandmaterial der eingangs genannten Art ist beispielsweise in der DE-AS 21 03 590 beschrieben.

Die wasserundurchlässige Trägerschicht ist dabei definiert als "ein flexibles oder verformbares mikroporöses, neutrales Folien- oder Gewebematerial", das undurchlässig für Flüssigkeiten und Bakterien und durchlässig für Gas ist. Die flüssigkeitsaufnehmende Schicht ist definiert als "irgendein neutrales, synthetisches, netzartiges, offenzelliges, festes Schaum- oder Schwammaterial", das der "Aufnahme von Zelltrümmern und flüssigem Exsudat" dienen soll.

Ein Verbandmaterial mit einer gasdurchlässigen, wasserdurchlässigen mikroporösen Kunststoffträgerschicht und einer flüssigkeitsaufnehmenden Schicht ist weiterhin aus einer Firmenschrift der Fa. Parke-Davis bekannt. Die Oberseite dieses Verbandmaterials besteht aus Polytetrafluorethylenfolie und die Unterseite aus Polyurethan-Schaumstoff.

In der DE-OS 31 11 336 wird ein Verbandmaterial mit einer der Wunde zugewandten Abdeckschicht aus Polytetrafluorethylen (PTFE) beschrieben.

In der europäischen Patentanmeldung 184 392 wird ein wasserundurchlässiges, wasserdampfdurchlässiges Material und ein Verfahren zu seiner Herstellung offenbart. In diesem Dokument ist ganz allgemein erwähnt, daß das erfindungsgemäße Material auch medizinischen Anwendungszwecken dienen könnte, ohne daß jedoch weiter auf Aufbau, Beschaffenheit oder Verwendung eingegangen wird.

Aufgabe der vorliegenden Erfindung war es, ein Verbandmaterial der eingangs genannten Art zur Verfügung zu stellen, das nicht nur die Wunde optimal schützt und gegebenenfalls mit Sauerstoff versorgt, sondern das darüberhinaus Schmerzen in den darunterliegenden Körperteilen lindert.

Diese Aufgabe wird bei einem Verbandmaterial der eingangs genannten Art dadurch gelöst, daß die flüssigkeitsaufnehmende Schicht (2) mit Wasser oder einer wäßrigen Lösung getränkt ist.

Ein solches Verbandmaterial hat im Vergleich zu den bisher handelsüblichen Verbandmaterialien den wesentlichen Vorteil, daß durch die gasdurchlässige Membran ein Luftaustausch zwischen dem durch das Verbandmaterial abgedecktem Körperbereich und der Umgebung stattfinden kann.

Erfindungsgemäß ist die flüssigkeitsaufnehmende Schicht (2) des Verbandmaterials bereits mit Flüssigkeit getränkt. "Tränken" bedeutet dabei, daß das flüssigkeitsaufnehmende Material "möglichst viel Flüssigkeit aufgenommen" haben soll (Duden, Bedeutungswörterbuch, Bibliographisches Institut Mannheim, Wien, Zürich, 1970). Zwar sind aus dem DE-GM 258 336 bereits feuchte Dauerkompressen bekannt, jedoch handelt es sich dabei um ein zweischichtiges Verbandmaterial, dessen äußere Schicht feuchtigkeits- und luftdurchlässig ist. Dies führt im abgedeckten Körperbereich zu einer feuchten Kammer, die z.B. für die Heilung angegriffener Haut überaus schädlich ist. Das erfindungsgemäße Verbandmaterial hingegen kann je nach Art der applizierten Flüssigkeit vorteilhaft für die Behandlung z.B. von Schürf- oder Brandwunden eingesetzt werden; darüberhinaus erfüllt es auch die Funktionen eines feuchten Umschlages, z.B. bei der Behandlung von entzündlichen Prozessen, bei der Schmerzlinderung von durch Hämatomen oder anderen Verletzungen verursachten Beschwerden usw.. Ein besonderer Vorteil besteht in diesem Zusammenhang darin, daß durch die Mikroporösität der Membran ein ständiger Temperaturaustausch zwischen abgedecktem Bereich und Außenmilieu stattfindet. Dadurch wird eine Überwärmung des abgedeckten Bereiches vermieden und eine wohltuende, gleichmäßige Kühlung auf Raumtemperatur erreicht.

Die flüssïgkeitsaufnehmende Schicht (2) kann beïspielsweise mit einer Lösung von essigsaurer Tonerde, Kamillen- oder Arnikaextrakten oder Lösungen antibiotisch oder antimykotisch wirkenden Substanzen getränkt werden. Letzteres ist vor allem bei der Behandlung von offener Wunden oder von Brandverletzungen vorteilhaft.

Bevorzugt ist die Verwendung von reinem, Insbesondere von destilliertem oder sterilisiertem Wasser. Es hat sich nämlich gezeigt, daß bei Verwendung von erfindungsgemäßen, wassergetränkten Verbandmaterialien eine überraschende Wirkung in einer unerwartet weitgehenden Schmerzlinderung besteht. So wurden in mehreren Fällen durch Gastritiden hervorgerufene Schmerzen durch Anwendung eines erfindungsgemäßen wassergetränkten Verbandes vollkommen beseitigt. Die überraschende schmerzlindernde bzw. -beseitigende Wirkung des wassergetränkten Verbandes erwies sich außerdem bei der Behandlung von operationsbedingten Schwellungen und der Schmerzen im Operationsgebiet. Eine weitere, vollkommen unerwartete Wirksamkeit wurde bei der Behandlung von Verbrennungen zweiten bis dritten Grades beobachtet; in diesem Fall waren bei sofortiger Anwendung eines wassergetränkten erfindungsgemäßen Verbandes völlige Schmerzfreiheit sowie eine überraschend schnelle Heilung des vollkommen reizlosen Wundgebietes zu beobachten.

Die Kunststoffträgerschicht (1) sollte bevorzugt eine Porengröße von 0,45 bis 2 µm, bevorzugt 1 µm, aufweisen. Diese Porengröße gestattet einen freien Durchtritt von Gasen, während tropfenförmige Feuchtigkeit aufgrund der zu hohen Oberflächenspannung nicht durchtritt. Da die erfindungsgemäße Kunststoffträgerschicht (1) darüber hinaus eine gewisse Flexibilität aufweisen soll, um sich im jeweiligen Anwendungsbereich an die Körperkonturen anpassen zu können, hat sie eine Dicke von 0,4 bis 1 mm, bevorzugt 0,55 bis 0,65 mm.

Das Verbandmaterial kann aus jeder beliebigen wasserundurchlässigen Kunststoffträgerschicht, die für Gas und Wasserdampf durchlässig und mikroporös ist, sowie einer beliebigen flüssigkeitsaufnehmenden Schicht zusammengesetzt sein. Bevorzugt ist es jedoch, als Kunststoffträgerschicht (1) eine Schicht aus Polytetrafluorethylen zu verwenden. Besonders bevorzugt sind dabei Materialien, die aus gerecktem Polytetrafluorethylen bestehen und eine gleichmäßige Verteilung ungefähr gleich großer Poren gewährleisten.

Eine vorteilhafte Eigenschaft des Verbandmaterials ist es, daß bei seiner Anwendung auch auf der Haut empfindlicher Menschen keine Abwehrreaktionen hervorgerufen werden. Das erfindungsgemäße Material zeichnet sich durch eine hohe Bïokompatibilität aus. Darüber hinaus ist es reißfest, was besonders bei der Anwendung an Stellen, an denen es gewissen Belastungen ausgesetzt ist, beispielsweise an Gelenken, sinnvoll ist.

Die flüssigkeitsaufnehmende Schicht (2) des erfindungsgemäßen Verbandmaterials kann jede beliebige aus dem Stand der Technik bekannte flüssigkeitsaufnehmende Schicht sein. Beispiele für solche flüssigkeitsaufnehmenden Schichten sind z.B. Zellstoffmullschichten oder Wattemullschichten.

Das erflndungsgemäße Verbandmaterlal wird in einer weiteren bevorzugten Ausführungsform in Form einer selbstklebenden Abdeckung zur Verfügung gestellt. Diese Ausführungsform sieht vor, daß die wasserundurchlässige Kunststoffträgerschicht (1) an zwei oder mehr Seiten über die flüssigkeitsaufnehmende Schicht (2) übersteht, wobei die überstehenden Flächen der Kunststoffträgerschicht mit einem hautverträglichen Klebemittel (3) beschichtet sind. Ein so vorbereitetes Verbandmaterial ist daher selbstklebend. Als hautverträgliche Klebemittel können übliche Klebemittel verwendet werden, z.B. eine Mischung aus Naturkautschuk, Zinkoxid, Harz und Lanolin oder aus Phtalatharz, Polyvinyletherdïspersionen und Acrylatmischpolymerisaten. Jedes andere hautverträgliche Klebemittel kann ebenso verwendet werden. Darüberhinaus besteht die Möglichkeit, das erfindungsgemäße Verbandmaterial durch gebräuchliche Pflasterstreifen oder Verbände auf der Haut zu befestigen.

Bei der Behandlung von inneren entzündlichen Prozessen, z.B. Gastritiden, oder bei der Kühlung von schmerzhaften Schwellungen ist es nicht erforderlich, daß das Verbandmaterial steril ist. Für die Behandlung offener Wunden oder Brandwunden ist jedoch die Bereitstellung steril verpackten erfindungsgemäßen Verbandmaterials bevorzugt.

Das erfindungsgemäße Verbandmaterlal kommt in sehr unterschiedlichen Größen zur Anwendung. Für die Versorgung einer kleinen Schnittwunde oder einer punktuellen Verbrennung reichen Verbände, deren feuchtigkeitsaufnehmende Schicht eine Größe von 0,25 bis 1 cm² haben und auch in Kreisform vorliegen können; für die Schmerzlinderung z.B. bei einer Gastritis sind hingegen Verbände nützlich, deren feuchtigkeitsaufnehmende Schicht die gesamte Bauchfläche abdecken. Die Breite der überstehenden Fläche der Kunststoffträgerschicht entspricht dabei jeweils den Bedürfnissen und beträgt beispielsweise bei den oben erwähnten kleinen runden Verbänden ca. 0,5 bis 1 cm, bei größeren Verbänden jedoch 15, 20 in Extremfällen sogar 30 cm oder mehr.

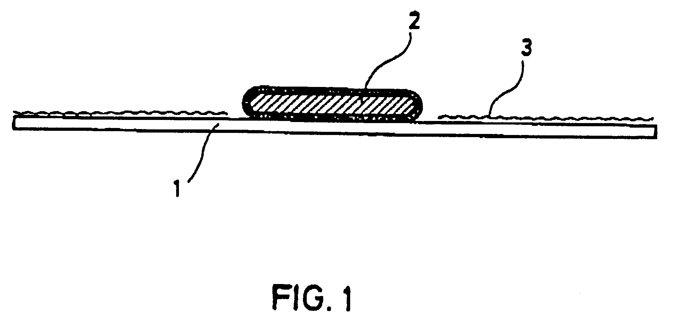

Die Erfindung wird nachstehend anhand eines Ausführungsbeispieles und einer dazugehörigen Zeichnung näher erläutert. Dabei zeigt die Figur eine Ausführungsform des erfindungsgemäßen Verbandmaterials im Querschnitt. In dieser Figur bedeutet:
(1) eine wasserdampfdurchlässige mikroporöse Kunststoff trägerschicht,
(2) die auf (1) aufgebrachte flüssigkeitsaufnehmende Schicht (in dieser Zeichnung handelt es sich dabei um eine vliesummantelte Mullschicht),
(3) eine dünne Schicht eines Klebemittels.

### Beispiel

Eine unter ärztlicher Aufsicht bewußt durch Auflegen eines auf 800 bis 900°C erhitzten Metallbandes von ca. 0,8 x 2,5 cm verursachte Brandwunde wurde mit einem erfindungsgemäßen Verband versorgt, bei dem die feuchtigkeitsaufnehmende Schicht aus mit destilliertem Wasser getränkter Verbandsgaze bestand. Auch nach Nachlassen der vor Durchführen der Verbrennung applizierten Lokalanästhesie waren keine Schmerzen spürbar. Das Verbandmaterial wurde nach 17 h entfernt und die vollkommen reizlose Wunde weitere 24 h mit einem normalen Pflaster geschützt. Nach belauf dieser 24 h war die Wunde nicht weiter schutzbedürftig.

## Patentansprüche

1. Verbandmaterial mit einer für Gas und Wasserdampf durchlässigen, für Wasser undurchlässigen mikroporösen Kunststoffträgerschicht (1) und einer darauf aufgebrachten flüssigkeitsaufnehmenden Schicht (2), dadurch gekennzeichnet, daß die flüssigkeitsaufnehmende Schicht (2) mit Wasser oder einer wäßrigen Lösung getränkt ist.

2. Verbandmaterial nach Anspruch 1, dadurch gekennzeichnet, daß die Kunststoffträgerschicht um eine Porengröße von 0,45 bis 2 µm, bevorzugt 1 µm aufweist und eine Dicke von 0,4 bis 1 mm, bevorzugt 0,55 bis 0,65 mm, hat.

3. Verbandmaterial nach Anspruch 1 und/oder 2, dadurch gekennzeichnet, daß die Kunststoffträgerschicht (1) aus gerecktem Polytetrafluorethylen besteht.

4. Verbandmaterial nach mindestens einem der Ansprüche 1 bis 3, dadurch gekennzeichnet, daß die Kunststoffträgerschicht (1) biokompatibel und/oder reißfest ist.

5. Verbandmaterial nach mindestens einem der Ansprüche 1 bis 4, dadurch gekennzeichnet, daß die flüssigkeitsaufnehmende Schicht (2) eine Zellstoff- Mullschicht oder eine Watte-Mullschicht oder eine Schicht aus vliesummanteltem Mull ist.

6. Verbandmaterial nach mindestens einem der Ansprüche 1 bis 5, dadurch gekennzeichnet, daß die wasserundurchlässige Schicht (2) übersteht und diese überstehende Fläche mit einem hautverträglichen Klebemittel (3) beschichtet ist.

7. Verbandmaterial nach mindestens einem der Ansprüche 1 bis 6, dadurch gekennzeichnet, daß es steril verpackt ist.

## Claims

1. A bandaging material having a microporous plastic carrier layer (1) which is permeable to gas and water vapor and impermeable to water and a liquid-absorbing layer (2) being positioned thereon, wherein the liquid-absorbing layer (2) is impregnated with water or an aqueous solution

2. The bandaging material according to claim 1 wherein the plastic carrier layer has a pore size of 0.45 to 2 µm, preferably 1 µm, and a thickness of 0.4 to 1 mm, preferably 0.55 to 0.65 mm.

3. The bandaging material according to claim 1 and/or 2 wherein the plastic carrier layer (1) consists of stretched polytetrafluoroethylene.

4. The bandaging material according to at least one of claims 1 to 3 wherein the plastic carrier layer (1) is biocompatible and/or resistant to tear.

5. The bandaging material according to at least one of claims 1 to 4 wherein the liquid-absorbing layer (2) is a gauze layer of cellulose or cotton wool, or a gauze layer encased with a non-woven.

6. The bandaging material according to at least one of claims 1 to 5 wherein the water impermeable layer (2) projects, and said projecting area is coated with an adhesive (3) which is well tolerated by the skin.

7. The bandaging material according to at least one of claims 1 to 6 in aseptically packed form.

## Revendications

1. Matériau pour pansement à couche de base (1) en plastique microporeux, perméable aux gaz et à la vapeur d'eau et imperméable à l'eau et, posée sur celle-ci, une couche hydrophile (2), caractérisé en ce que ladite couche hydrophile (2) est imprégnée d'eau ou d'une solution aqueuse.

2. Matériau pour pansement selon la revendication 1, caractérisé en ce que la couche de base (1) en plastique présente des pores d'une dimension de 0,45 à 2 µm, et de préférence de 1 µm, et a une épaisseur de 0,4 à 1 mm, et de préférence de 0,55 à O,65 mm.

3. Matériau pour pansement selon les revendications 1 et/ou 2, caractérisé en ce que la couche de base (1) en plastique est constituée de polytétrafluoroéthylène étiré.

4. Matériau pour pansement selon au moins une des revendications de 1 à 3, caractérisé en ce que la couche de base (1) en plastique est biocompatible et/ou résistante aux déchirures.

5. Matériau pour pansement selon au moins une des revendications de 1 à 4, caractérisé en ce que la couche hydrophile (2) est constituée d'une couche en gaze cellulosique, ou d'une couche en gaze d'ouate, ou par une couche de gaze enveloppée dans un feutre.

6. Matériau pour pansement selon au moins une des revendications de 1 à 5, caractérisé en ce que la couche imperméable (2) déborde et que cette surface débordante est enduite d'un agent adhésif (3) neutre pour la peau.

7. Matériau pour pansement selon au moins une des revendications de 1 à 6, caractérisé en ce que son emballage est stérile.
